# EUROPEAN PATENT APPLICATION

(11) **EP 2 623 127 A1**
(43) Date of publication of application: **07.08.2013**
(21) Application number: 13154116.1
(22) Date of filing: 06.02.2013
(51) Int. Cl.: A61L 2/24, A61L 2/26, A47L 15/00

(54) **Washer machine**

(30) Priority: 06.02.2012 IT UD20120018
(71) Applicant: STEELCO SPA, 31039 Riese Pio X (IT)
(72) Inventor: Zardini, Fabio, 31033 Castelfranco Veneto (TV) (IT)
(74) Representative: Petraz, Davide Luigi

(57) **Abstract**

Washer machine (10) for objects, comprises an inner washing chamber (14), accessed through an aperture (16) associated with a closing device (22) comprising a door (24) cooperating with peripheral edges (20) that delimit said aperture (16). The closing device (22) comprises a plurality of pneumatic gaskets (26, 28), each of which is connected to its own pressurized pneumatic means (30, 32), operatively independent of each other and able to introduce a pressurized fluid inside the respective gaskets (26, 28), selectively determining a dilated operating condition thereof to achieve the hydraulic seal.

## Description

### FIELD OF THE INVENTION

The present invention concerns a washer machine provided with hydraulic seal gaskets. In particular, the present invention concerns a machine for washing objects, including both the pre-treatment of the objects, such as a pre-wash with hot or cold water and/or with detergents or other chemical products, and also a real and proper wash, but also heat disinfection and/or sterilization, in particular also decontamination using particularly aggressive and dangerous decontaminating substances such as oxygenated water. By way of example, the objects which can be washed in the machine in question can be laboratory instruments, for research, instruments used in the pharmaceutical sector, or instruments in the medical sector, surgical instruments or similar or comparable instruments, without however excluding the application of the present invention to washing objects in general.

### BACKGROUND OF THE INVENTION

Washer machines are known, with a washing chamber which can be accessed through an aperture, typically at the front, so that the objects to be washed can be inserted through it, and provided with a closing device formed by a hinged door, hinged along one of the peripheral edges which delimit the aperture, which cooperates with the peripheral edges of the aperture.

The closing device is provided with a single hollow gasket, generally made of rubber or similar elastomeric material, for the hydraulic seal.

The gasket is disposed along a continuous seating made over the whole external perimeter of the hinged door.

The gasket is the pneumatic type, being associated to a pressurized pneumatic device, such as a compressed air connection device, configured for introducing a fluid, typically air, compressed inside the gasket, so as to define a first dilated operating condition of the gasket which, when the hinged door is closed, ensures the hydraulic seal cooperating with the peripheral edges of the aperture. Once the compressed air has been released, the dilated operating condition is reversible in a substantially elastic way, moving into a second inactive position, when it is necessary to open the door.

Document US-A-2003/168080 describes a known dishwasher which includes a door and a control circuit connected to a pump or sprayer and to an inflatable gasket.

Documents AU-B-756375 and WO-A-01/26532 describe a known dishwasher with a lid associated to an inflatable gasket.

A known dishwasher with a lid and an inflatable gasket is also described in US-B-6,460,555.

However, one disadvantage of known closing devices is that, in the case of a malfunction of the pressurized pneumatic device, or of damage to or perforation of the gasket, the gasket is not able to assume the first dilated operating condition. This has the effect that, during the washing cycle, there are leakages of dangerous or toxic substances, detergents or aggressive chemical products, toward the external working environment, with safety risks for the operators, as well as pollution for the environment.

A pressure sensor associated to the gasket can be provided, in order to control the actual internal pressure, as can a pressure sensor associated to the chamber, which controls the pressure inside the latter. Thus, in the case of malfunction or loss of pressure, an alarm signal can be sent. However, such strategies using sensors do not prevent that, during functioning and in the event of leakages, there is a considerable safety risk for the operators and pollution in the work environment. In any case, the pressure sensors, in particular the one associated to the gasket, do not in themselves guarantee the hydraulic seal, which can also be compromised by other factors, such as, for example, the deformation of the gasket seating, or the thrust zone of the gasket itself.

There is therefore the need to perfect a washer machine which can overcome at least one of the disadvantages of the state of the art.

In particular, one purpose of the present invention is to obtain a washer machine which has a closing device that guarantees the hydraulic seal, and therefore the safety, even in the case of malfunction, breakage or damage of the hydraulic gasket.

Another purpose is to ensure the closing device is easy and economic to produce.

The Applicant has devised, tested and embodied the present invention to overcome the shortcomings of the state of the art and to obtain these and other purposes and advantages.

### SUMMARY OF THE INVENTION

The present invention is set forth and characterized in the independent claims, while the dependent claims describe other characteristics of the invention or variants to the main inventive idea.

In accordance with the above purposes, a washer machine for objects according to the present invention comprises an internal washing chamber, which is accessed through at least one aperture delimited by peripheral edges, and a closing device comprising at least one door for the aperture, in particular in order to close in abutment on defined thrust zones typically represented by the peripheral edges of the aperture.

According to aspects of the present invention, the closing device comprises:
- a plurality of pneumatic gaskets disposed adjacent and in series with respect to each other inside mating seatings, to obtain a hydraulic seal between the door and the thrust zones represented by the peripheral edges of the aperture,
- a plurality of pressurized pneumatic devices, such as a connection device with compressed air or other compressed fluid, operatively independent from each other and configured to introduce a pressurized fluid, typically compressed air, inside the gaskets, selectively determining a dilated operating condition in order to obtain the hydraulic seal,
in which each of the gaskets is connected to its own and corresponding one of the pressurized pneumatic devices.

Thanks to the plurality of independent pneumatic gaskets, the invention advantageously obtains a redundancy in safety for the purposes of the hydraulic seal since, if one of the gaskets or the corresponding pressurized pneumatic device is damaged, inactive or in any case does not give the necessary dilated operating condition, or if one of the seatings for the gaskets or the corresponding thrust zones is deformed, the hydraulic seal will in any case be guaranteed by the remaining gaskets and by the associated pressurized pneumatic devices.

According to some embodiments the machine in question comprises at least one containing wall disposed between adjacent gaskets.

According to some embodiments, the at least one containing wall is continuous and disposed between the gaskets, completely following the whole development or profile of the gaskets, to act as a physical barrier between them. In this way, if one of the gaskets is not dilated, the corresponding housing space will not be occupied by the one or more adjacent dilated gaskets.

In some embodiments, the gaskets are mounted on the door.

In some embodiments, the seatings are made on the door.

In other embodiments, the gaskets are mounted along the peripheral edges of the aperture.

In some embodiments, the seatings are made along the peripheral edges of the aperture.

In some embodiments, the machine comprises one or more gaskets mounted on the door and one or more gaskets mounted along the peripheral edges of the aperture.

In some embodiments, at least one of the gaskets, or some or even all of the gaskets, are provided with one or more friction elements protruding from their surface.

In some embodiments, the pressurized pneumatic devices are connected to the corresponding gaskets by means of flexible connection pipes.

In some embodiments, the gaskets are made of elastomeric material.

In some embodiments, the seatings are made as grooves, in particular with a concave cross section.

In some embodiments, the gaskets and the seatings are reciprocally sized so that, in the dilated operating condition, the gaskets protrude from the seatings.

In some embodiments, the machine can possibly signal automatically, by means of a system controller, any malfunctions or defects in the gaskets or the pneumatic system.

According to some aspects described herein, a method may be provided to control the safe closing of a washer machine as described above, which includes verification of the efficiency of the gaskets by detecting the pressure of the compressed fluid in the gaskets and, if a signal, generated by said detection, and interpreted by a system controller, corresponds to a pressure value outside a predefined operating pressure range, the system controller transmits an automatic warning signal or alarm.

In some embodiments, a washing machine is provided as described above, comprising a system controller and a computer program memorizable in a memory readable by a computer that contains the instructions which, when executed by the system controller, determine the execution of a method to control the safe closing of a washer machine as described herein.

### BRIEF DESCRIPTION OF THE DRAWINGS

These and other characteristics of the present invention will become apparent from the following description of some embodiments, given as a non-restrictive example with reference to the attached drawings wherein:
- fig. 1 is a perspective view of a washer machine according to the present invention in an open door condition;
- fig. 2 is a lateral view of the door in fig. 1;
- fig. 3 is an enlarged detail of fig. 2;
- fig. 4 is a variant of the machine in fig. 1;
- fig. 5 is another variant of the machine in fig. 1.

To facilitate comprehension, the same reference numbers have been used, where possible, to identify identical common elements in the drawings. It is understood that elements and characteristics of one embodiment can conveniently be incorporated into other embodiments without further clarifications.

### DETAILED DESCRIPTION OF SOME EMBODIMENTS

We shall now refer in detail to various embodiments of the present invention, of which one or more examples are shown in the attached drawings. Generally, only the differences from the individual embodiments will be described. Each example is supplied to illustrate the present invention and shall not be understood as a limitation to the present invention. For example, the characteristics shown or described as part of one embodiment can be adopted in or in association with other embodiments to produce another embodiment. It is understood that the present invention shall include all such modifications and variants.

With reference to the attached drawings, the reference number 10 denotes in its entirety a washer machine according to possible embodiments of the present invention described herein, used for example but not restrictively for a pre-washing treatment, with hot or cold water and/or detergents or other chemical products, or the washing treatment proper, or also heat disinfection and/or sterilization of objects, such as for example surgical instruments, or laboratory instruments or similar or comparable instruments.

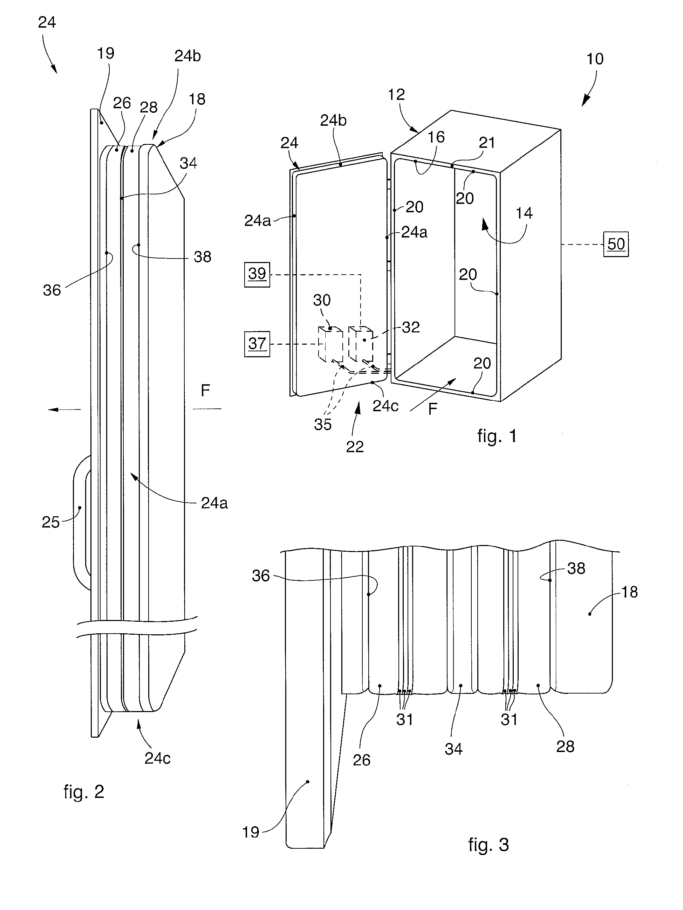

Fig. 1 is used to describe a plurality of embodiments, which can be combined with all the other embodiments described herein, in which the machine 10 comprises an external casing 12 which can delimit an internal washing chamber 14, accessed for example through at least one aperture 16, in this case made at the front, used for the manual or automatic loading or insertion of the objects to be washed, in random or organized form and disposed in racks or similar containers, the loading being performed generally in a direction of loading, indicated by the letter F in fig. 1, which is transverse, for example orthogonal, to the plane on which the aperture 16 lies.

Although a machine 10 is described herein with a single front aperture 16, there is nothing to prevent the present invention from being applied also to machines with apertures made differently, such as lateral, or upper, and also to machines 10 with a double aperture, known as "pass-through" machines.

In possible example of implementation, the aperture 16 can be delimited on the perimeter by peripheral edges 20. The shape of the aperture 16 is shown in the drawings with a rectangular shape, but other shapes are not excluded, such as polygonal, circular or elliptical.

In some embodiments, described with reference for example to figs. 1, 4 and 5, the machine 10 comprises a closing device 22 configured to close the aperture 16, for example during the washing cycle, so as to guarantee the hydraulic seal and prevent leaks, leakages or spills of liquids or other dangerous, contaminating or aggressive gases exiting from the chamber 14 during use.

In possible implementations, the closing device 22 comprises at least one door 24, selectively mobile between an open condition (figs. 1, 4 and 5) and a closed condition in which it cooperates directly with the aperture 16, or closes the aperture 16, in particular abutting with the peripheral edges 20 that function as thrust zones of the door 24.

Fig. 2 is used to describe possible embodiments, which can be combined with all the embodiments described herein, in which the door 24 is, in the example shown, a hinged door, which can be hinged laterally in proximity to one of the peripheral edges 20 of the aperture 16 and provided with a handle 25 (fig. 2). The door 24 can be rather big, for example with a height of about two meters, width of about one meter and a thickness of some centimeters.

In possible implementations, with reference to fig. 2 for example, the door 24 comprises for example a closing body 18, with a shape mating with that of the aperture 16 in order to close it, which can have on the perimeter abutment fins 19 that are configured to cooperate for example with an external abutment band 21 of the aperture 16.

In possible implementations, the closing body 18 can be covered by a metal frame, useful for example to support the physical and chemical conditions of the chamber 14 during the washing cycle.

Furthermore, according to possible embodiments, which can be combined with all the embodiments described herein, the closing device 22 can comprise a plurality of pneumatic gaskets 26, 28, in the case shown here two gaskets 26, 28, however the possibility of two or more gaskets 26, 28 is not excluded, for example three gaskets or even more. For the purposes of the safety of the hydraulic seal, the gaskets 26, 28 can be activated and/or de-activated independently of each other, for example they can be inflated or deflated autonomously, in sequence with respect to each other or simultaneously.

In some embodiments, the gaskets 26, 28 can be configured to affect, autonomously from each other, all the peripheral edges 20 of the aperture 16, thus guaranteeing the hydraulic seal when the door 14 is closed. In other words, the gaskets 26, 28 can extend for the whole extension of the peripheral edges 20 of the aperture 16.

In possible embodiments, the closing body 18 and the mating peripheral edges 20 of the aperture 16 can for example be provided with the tops rounded (fig. 2), so as to facilitate the thrust action of the gaskets 26, 28 and make it uniform.

Each of the gaskets 26, 28 can be fluidically connected to its own pressurized pneumatic devices 30, 32, such as for example a connection device with the introduction of compressed air, or a compressed air circuit, or similar compressed fluid.

In possible implementations, the pressurized pneumatic devices 30, 32 can comprise the feed of a fluid, such as air, a pump to increase the pressure of the fluid received from the feed, a circuit, for example formed by one or more pipes, to transport the compressed fluid from the delivery pipe of the pump to the gaskets 26, 28, and connectors to introduce the compressed fluid into the gaskets 26, 28. Possibly, the pressurized pneumatic devices 30, 32 can also be equipped with one or more valves to control or regulate the flow rate of the fluid, and/or pressure sensors and/or set point controllers or other mechanical, electromechanical, electric or electronic control and/or adjustment components.

The pressurized pneumatic devices 30, 32 are configured to introduce air or other suitable fluid under pressure inside the cavities of the respective fluidically associated gaskets 26, 28, in order to inflate them. Furthermore, the pressurized pneumatic devices 30, 32 can be configured so that, once de-activated, the pressure inside the gaskets 26, 28 is released, thus causing them to deflate. The pressurized pneumatic devices 30, 32, shown only schematically in figs. 1, 4 and 5 by way of example, can be integrated into the machine 10, or outside it.

The selective introduction of the pressurized fluid into the gaskets 26, 28 by the pressurized pneumatic devices 30, 32 can be activated once the door 24 is closed. The introduction of the pressurized fluid determines a dilated or inflated operating condition of the gasket 26, 28 so that as they increase in volume they can cooperate under compression, in particular they can thrust in abutment, with the peripheral edges 20 of the aperture 16 and/or with the door 24, according to the possible variant embodiments, as will be explained in more detail hereafter, and thus guarantee the hydraulic seal. According to the present invention, once the door 24 is closed, both the gaskets 26, 28 are dilated or inflated as explained above, simultaneously or in sequence.

According to the present invention, when the door 24 is closed, the gaskets 26, 28 are disposed adjacent and in series or succession along the loading direction F, thus guaranteeing the seal from inside to outside. In other words, the gaskets 26, 28 lie on corresponding lying planes that are disposed in sequence parallel to each other and parallel to the lying plane of the aperture 16 and/or the door 24. In particular, in embodiments where the gaskets 26, 28 are disposed along the peripheral edges 20 of the aperture 16, the lying planes of the gaskets 26, 28 are transverse, for example perpendicular, to the loading direction F. In embodiments where the gaskets 26, 28 are disposed along edges of the door 24, the lying planes of the gaskets 26, 28 are transverse, for example perpendicular, to the loading direction F when the door 24 is closed.

During possible operations when it is necessary to open the door 24, for example at end-of-cycle, or in the event of extraordinary interventions, the pressurized pneumatic devices 30, 32 are configured to release the pressure of the compressed fluid present in the gaskets 26, 28, in this case too either simultaneously or in sequence with each other, so that the gaskets 26, 28 are in a second inactive condition, not dilated, or deflated, in which they do not cooperate under compression, that is, they do not thrust in abutment nor do they substantially interfere with the peripheral edges 20 of the aperture 16. In some embodiments, the gaskets 26, 28 can be made of elastomeric material or similar. For example, the second inactive condition of the gaskets 26, 28 can be obtained in a substantially reversible manner, also thanks to the elastomeric material or similar that makes up the gaskets 26, 28.

The pressurized pneumatic devices 30, 32 can be operatively independent from each other, that is to say, they are configured to be used autonomously from each other in order to inflate and deflate the respective gaskets 26, 28.

Consequently, in the event of a malfunction of one of the pressurized pneumatic devices 30, 32, or the breakage, perforation or other damage or defect in one of the gaskets 26, 28, the other gasket or gaskets 26, 28 that remains/remain operative can in any case be kept in an inflated condition and therefore can perform the hydraulic seal functions and hence guarantee the safety of the machine 10 during the washing cycle, preventing potentially dangerous, toxic or polluting leaks, leakages or spills and allowing, in any case, to use the machine without causing it to stop.

In some embodiments, each of the gaskets 26, 28 can be configured to perform the hydraulic seal functions required autonomously, reliably and safely in its dilated operating condition. Consequently, in the event of a malfunction of one gasket 26, 28, the other gasket 26, 28 is in any case able to guarantee the hydraulic seal.

According to some embodiments, which can be combined with all the embodiments described herein, housing seatings 36, 38 are provided, inside which the gaskets 26, 28 are disposed.

For example, the seatings 36, 38 can be made as continuous grooves, for example with a concave cross section, for example substantially U-shaped.

The gaskets 26, 28 and the seatings 36, 38 can be reciprocally sized so that, in the dilated operating condition, the gaskets 26, 28 protrude adequately by a desired amount from the seatings 36, 38 so as to obtain the hydraulic seal with the door 24 closed, whereas in the inactive condition they are for example at least flush with, or included in, the bulk of the seatings 36, 38, that is, they do not protrude from them, so they do not constitute an obstacle to the movement of the door 24.

According to some embodiments of the present invention, a containing wall or platen 34 may be provided, disposed between the gaskets 26, 28, for example separating one seating 36 from the other seating 38. In embodiments where more than two gaskets 26, 28 are provided, a containing wall or platen 34 may be disposed between each pair of adjacent gaskets 26, 28.

The containing wall or platen 34 may be configured continuous, extending along the whole development of the gaskets 26, 28 and of the seatings 36, 38. In this way, since the containing wall or platen 34 is disposed intermediate between the gaskets 26, 28, and completely follows the whole development or profile of the gaskets 26, 28, it functions as a physical barrier between the latter. Considering the door 24 in the closed condition, the containing wall or platen 34, is provided between one gasket 26, 28 and the adjacent one, that is, between at least one pair of adjacent gaskets 26, 28.

This can have the advantage that, if one of the gaskets 26, 28 is not dilated following a malfunction or damage, the other gasket 26, 28, active and therefore dilated and disposed adjacent, does not invade the housing space of the adjacent non-dilated gasket 26, 28.

In some embodiments, the gaskets 26, 28 can all be mounted exclusively on the door 24, or all mounted exclusively along the peripheral edges 20 of the aperture 16 or, in other possible embodiments, partly mounted on the door 24 and partly mounted along the peripheral edges 20 of the aperture 16.

Figs. 1 - 3 are used to describe embodiments where two possible gaskets 26, 28 are both mounted on the door 24.

In particular, with reference for example to figs. 1 - 3, along the lateral edges 24a, upper edge 24b and lower edge 24c of the closing body 18, the door 24 may have a coordinated plurality of continuous seatings 36, 38, in this case for example two, so that each houses inside it a corresponding gasket 26, 28. The seatings 36, 38, made in series parallel to each other, can develop along the whole peripheral profile of the door 24.

Furthermore, according to some embodiments, each seating 36, 38 is separated from the one or more adjacent seatings 36, 38 by a corresponding one of the containing walls 34.

With reference to possible example embodiments, such as for example in figs. 1 - 3, a single containing wall 34 for example may be provided, which separates the two seatings 36, 38 in which the corresponding gaskets 26, 28 are housed.

In some embodiments, which can be combined with all the embodiments described herein, the one or more containing walls 34 possibly provided can for example be made in a single body with the door 24. In other embodiments, the one or more containing walls 34 possibly provided can for example be made as components separated from the door 24 and subsequently attached to it, for example by welding. The one or more containing walls 34 can be made of metal, or other similar materials, preferably with a suitable mechanical resistance to support the compression thrust of the gaskets 26, 28.

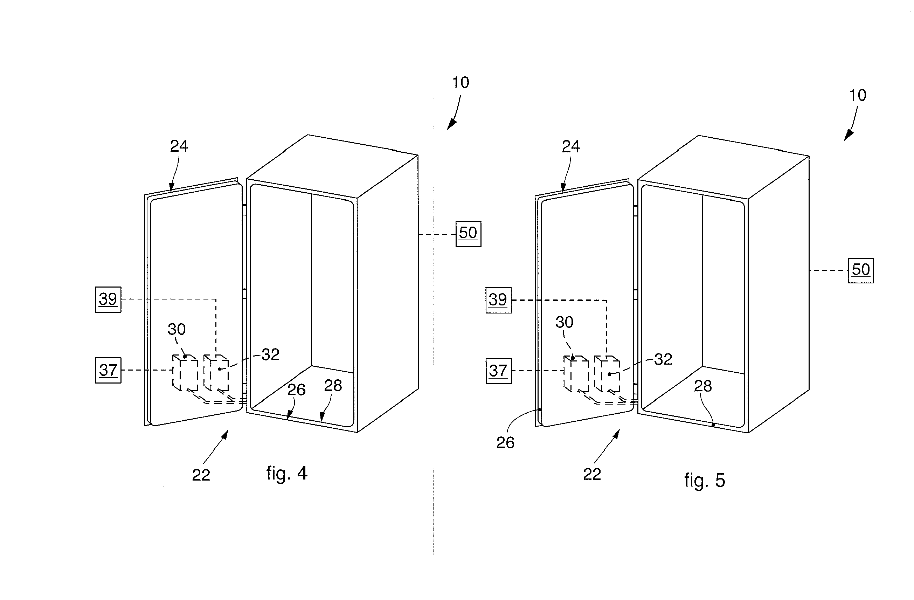

Fig. 4 is used to describe a plurality of embodiments, which can be combined with all the embodiments described herein, in which the two gaskets 26, 28 are both mounted along the peripheral edges 20 of the aperture 16. In this variant, both the seatings 36, 38 are made along the peripheral edges 20 of the aperture 16.

Fig. 5 is used to describe a plurality of embodiments, which can be combined with all the embodiments described herein, in which at least one gasket 26 is mounted on the door 24 and at least another gasket 28 is mounted along the peripheral edges 20 of the aperture 16. In some embodiments, at least one seating 36 may be made on the door 24, to accommodate a gasket 26, and at least another seating 38 made along the peripheral edges 20 of the aperture 16, to accommodate the other gasket 28.

As described above, also in the embodiments described using figs. 4 and 5, more than two gaskets 26, 28 could be provided, and therefore more than two seatings 36, 38.

With reference for example to the embodiments described using figs. 1 - 3, the pressurized pneumatic devices 30, 32 can be connected to the gaskets 26, 28 provided on the door 24 by means of flexible connection pipes 35, for example to adapt to the rotational movement of the door 24 as it opens and closes. A similar solution, suitably adapted, can be provided for example for the embodiments described with reference to fig. 4.

Furthermore, with reference for example to the embodiments described using figs. 1 - 3, which is valid also for the embodiments described using figs. 4 and 5, the gaskets 26, 28 can be configured with one or more friction elements 31 protruding from their surface, such as ridges, knurls or similar slightly protruding conformations, which develop along the profile of the gaskets 26, 28 and which can facilitate the hydraulic seal mechanically by achieving friction, that is, by functioning as gripping elements for the cooperation, that is, the closure, of the door 24 and the peripheral edges 20 of the aperture 16.

With the present invention it is possible to verify the efficiency of the gaskets 26, 28 by detecting the pressure of the fluid in the gaskets 26, 28 themselves, generally by means of a dedicated pressure sensor 37, 39 for each gasket 26, 28, typically but not restrictively incorporated, integrated or connected externally to the pressurized pneumatic devices 30, 32, which is configured to transmit to a system controller 50 a signal correlated to the value of the pressure of the compressed fluid present in the gaskets 26, 28. If this signal, interpreted by the system controller 50, corresponds to a pressure value outside a predefined range of operating pressure, for example below threshold pressure values, as can occur in the case of gaskets that are deflated, perforated or in some way damaged, the system controller 50 can be configured to transmit a corresponding automatic alarm signal.

If at least one of the remaining gaskets is in any case operative, the machine 10 can continue to work without any risk of leaking liquid toward the outside and, for example at the end of the operating cycle, an operator can verify the possible defective gasket that corresponds to the automatic alarm signal.

On the contrary, if the pressure inside more than one gasket, or in all the gaskets, is outside the pre-determined range, for example because more than one gasket is perforated or defective, the corresponding automatic alarm signal is used by the system controller 50 to modify the functioning of the machine 10 for safety reasons, which can for example cause the machine to stop, or at least the feed in the hydraulic circuit that supplies the washing liquid inside the machine to stop.

## Claims

1. Washer machine for objects, comprising an inner washing chamber (14), accessed through at least one aperture (16) delimited by peripheral edges (20), and a closing device (22) comprising at least one door (24) for said aperture (16), **characterized in that** said closing device (22) comprises:
- a plurality of pneumatic gaskets (26, 28) disposed adjacent and in series with respect to each other, inside mating seatings (36, 38) to achieve a hydraulic seal between said door (24) and the peripheral edges (20) of said aperture (16),
- a plurality of pressurized pneumatic devices (30, 32) operatively independent from each other and configured to introduce a fluid under pressure inside the gaskets (26, 28), selectively determining a dilated operating condition thereof in order to achieve the hydraulic seal, in which each of said gaskets (26, 28) is connected to its own and corresponding one of said pressurized pneumatic devices (30, 32).

2. Washer machine as in claim 1, **characterized in that** it comprises at least one containing wall (34) disposed between adjacent gaskets (26, 28).

3. Washer machine as in claim 2, **characterized in that** said at least one containing wall (34) is continuous and disposed between said gaskets (26, 28), completely following the whole development or profile of the gaskets (26, 28), to function as a physical barrier between them.

4. Washer machine as in any claim hereinbefore, **characterized in that** the gaskets (26, 28) are mounted on the door (24).

5. Washer machine as in claim 4, **characterized in that** the seatings (36, 38) are made on the door (24).

6. Washer machine as in any claim from 1 to 3, **characterized in that** the gaskets (26, 28) are mounted along the peripheral edges (20) of the aperture (16).

7. Washer machine as in claim 6, **characterized in that** the seatings (36, 38) are made along the peripheral edges (20) of the aperture (16).

8. Washer machine as in any claim from 1 to 3, **characterized in that** it comprises one or more gaskets (26, 28) mounted on the door (24) and one or more gaskets (26, 28) mounted along the peripheral edges (20) of the aperture (16).

9. Washer machine as in any claim hereinbefore, **characterized in that** at least one of the gaskets (26, 28) is provided with one or more friction elements (31) protruding from its surface.

10. Washer machine as in any claim hereinbefore, **characterized in that** the pressurized pneumatic devices (30, 32) are connected to the corresponding gaskets (26, 28) by means of flexible connection pipes (35).

11. Washer machine as in any claim hereinbefore, **characterized in that** the gaskets (26, 28) are made of elastomeric material.

12. Washer machine as in any claim hereinbefore, **characterized in that** the seatings (36, 38) are made as grooves, in particular with concave cross section.

13. Washer machine as in any claim hereinbefore, **characterized in that** the gaskets (26, 28) and the seatings (36, 38) are reciprocally sized so that, in the dilated operating condition, the gaskets (26, 28) protrude from the seatings (36, 38).

14. Washer machine as in any claim hereinbefore, configured for use in heat disinfecting washing and/or sterilization of objects.

15. Method to control the safe closing of a washer machine for objects as in any claim hereinbefore, **characterized in that** it provides the verification of the effectiveness of the gaskets (26, 28) by detecting the pressure of the compressed fluid present in the gaskets (26, 28) and if a signal generated by said detection, and interpreted by a system controller (50), corresponds to a pressure value outside a predefined operating pressure range, the system controller (50) transmits an automatic warning signal or alarm.

16. Washer machine as in any claim from 1 to 14, comprising a system controller (50) and a computer program memorizable in a memory readable by a computer which contains the instructions which, when executed by the system controller (50), determine the execution of a method as in claim 15.
